# EUROPEAN PATENT SPECIFICATION

(11) **EP 3 392 158 B1**
(45) Date of publication and mention of the grant of the patent: **04.08.2021**
(21) Application number: 18166397.2
(22) Date of filing: 09.04.2018
(51) Int. Cl.: B65B 55/10, B65B 55/16, B65B 61/20, A23C 3/07, A23L 2/46, A23L 2/48

(54) **PROCESS FOR ASEPTIC FILLING OF BEVERAGE PACKAGING COMPRISING AN INTERIOR DRINKING STRAW**
VERFAHREN ZUM ASEPTISCHEN FÜLLEN VON GETRÄNKEVERPACKUNGEN MIT INNENLIEGENDEM TRINKHALM
PROCÉDÉ DE REMPLISSAGE ASEPTIQUE D'EMBALLAGES DE BOISSONS COMPRENANT UNE PAILLE INTÉRIEURE

(30) Priority: 20.04.2017 GB 201706306
(43) Date of publication of application: 24.10.2018
(73) Proprietor: Deutsche Post AG, 53113 Bonn (DE)
(72) Inventor: Guru, Hari, West Midlands, WS1 2DH (GB); Michaels, Terry, Surrey, GU21 4NR (GB); George, Sam, Buckinghamshire, LU7 0LE (GB); Woodward, Simon, Northamptonshire, NN13 5HS (GB)
(74) Representative: Michalski Hüttermann & Partner Patentanwälte mbB

(56) References cited:
- US-A1- 2002 004 090
- US-A1- 2004 123 883
- US-B1- 6 405 764
- US-B2- 6 854 490

## Description

### FIELD OF THE INVENTION

The present invention relates to a cold filling process for the filling of beverages into a packaging comprising an interior drinking straw and a system for performing such process.

### BACKGROUND OF THE INVENTION

One key factor in the customers' perception of a product in the consumer goods sector is convenience. This statement is especially true for market situations, wherein supplier dependent quality differences of the good itself are difficult to detect or many alternatives or substitute products can be found. In such cases it is in most cases helpful to add secondary benefits to the product at hand for differentiation. Such strategy can for instance include a special packaging design, offering the consumer the possibility of a different and easier, i.e. more convenient, product use.

In the field of foodstuff such differentiation is also feasible, but the freedom in packaging design is, compared to other product classes, limited, because the most important task of the packaging is to guarantee the food safety over the defined minimum durability. This side-condition limits the overall amount of possible packaging solutions and restricts the choice to solutions only, which are able to guarantee microbiological safety.

Such considerations are especially important in the field of beverages. Beverages usually comprise high water activity, leading to excellent microbiological growth conditions. Hence, the products are very sensitive with respect to the packaging and the packaging process and alternative solutions comprising additional benefits with respect to the usability are rare.

One attempt to provide alternative drinking applications is the supply of a separate drinking straw to a beverage packaging. The straw provides a more hygienic and convenient drinking situation and is usually assembled in an independently packaged form at the outside of a beverage container. Due to the fact that both parts are individually packed it is possible to separate the production process and treat the microbiological side-conditions in processing and during storage of both parts independently.

Other technical solutions for beverages concern a situation, wherein a straw is included inside the beverage packaging.

US 6,854,490 B2 for instance disclose a flexible pouch with a self-contained straw for packaging a product, wherein the pouch includes a front panel and a back panel each having an upper edge, a lower edge opposite the upper edge, and side edges extending there between the upper and lower edges.

Another technical solution is described in EP 0 997 393 B1. In this patent document a packing sachet with a drinking straw or pouring tube arranged in the sachet and an easy-opening protected area as access to the straw or pouring tube is disclosed, where the sachet has a first compartment to hold a filling and a second compartment separated fluid-tight from the first by a sealing or adhesive seam and intended to hold a straw part.

Pouches including interior drinking straws are known in the literature. US 6,854,490 B2 for instance disclose a flexible pouch with a self-contained straw for packaging a product including a front panel and a back panel each having an upper edge, a lower edge opposite the upper edge, and side edges extending therebetween the upper and lower edges. A straw is disposed inside the pouch at an angle between an upper corner of the pouch and an opposite lower corner. The pouch includes a first seal applied to the upper corner of the pouch to form a pocket for holding an upper end of the straw, and a second seal extending along the unsealed portion of the upper edge of the pouch to close the pouch. The method of forming and filling a flexible pouch with an integral straw includes the steps of forming each of the panels and joining two panels by sealing together their side edges and lower edge to form the pouch. The method also includes the steps of opening the pouch and inserting the straw into the pouch by positioning the straw at an angle between an upper corner of the pouch and an opposite lower corner. The method further includes the steps of applying a first seal to the upper corner of the pouch to form a pocket for holding an upper end of the straw, and filling the pouch with the product. The method also includes the steps of applying a second seal extending along the unsealed portion of the upper edge of the pouch to close the pouch.

A process for packaging beverages into unmodified polyethylene terephthalate containers is disclosed in US 2002 0004 090 A1The process comprises: a) pasteurising the beverage, b) cooling the pasteurised beverage to a temperature below that at which the containers are deformed under the action of excessive heat, c) sterilisation of the containers and caps, d) filling of the sterilised receivers with cooled pasteurised beverage, e) sealing the filled sterilised containers, and f) further pasteurisation of the filled and sealed container at a temperature below that at which the containers deform under the action of excessive heat.

The integration of a straw in the beverage packaging generates a safer application, because the straw cannot unintentionally be separated from the beverage packaging. Unfortunately, the microbiological situation of the beverage changes completely, because the straw has to be fully considered in the microbiological background of the beverage and during beverage packaging. This severely limits the available packaging solutions and the suitable packaging processes. Based on safety consideration for the latter only hot-fill processes are state of the art. In such hot-fill processes the beverage is sterilized by heating up to temperatures suitable for killing microorganisms in the beverage and the heated beverage is also used to sterilize the beverage packaging and the included straw. Only the filling at high temperatures assures a clean microbiological background and sufficient shelf life. Unfortunately, this process is very energy-intensive, limited to batch processing and the thermal load on the beverage and the packaging is high, resulting in degradation of valuable ingredients or deterioration of the taste.

Therefore, it is the task of the present invention to disclose a packaging process and a system for performing such process, being able to overcome the shortcomings of the prior art and to provide a safe and economic packaging process, especially directed to the filling of beverage packaging containing an interior drinking straw.

### SUMMARY OF THE INVENTION

Above mentioned task is fulfilled by a process for filling of beverage packaging comprising an interior drinking straw, characterized in that the filling process at least comprises the steps of
a) In-line microwave assisted pasteurization of the beverages,
b) Vaporized Hydrogen Peroxide treatment of the packaging,
c) Vaporized Hydrogen Peroxide treatment of the straw,
d) Assembly of the Vaporized Hydrogen Peroxide treated straw in the Vaporized Hydrogen Peroxide treated packaging,
e) Fixation of the straw inside the packaging,
f) Temperature adjustment of the pasteurized beverage of step a) and cold filling of the pasteurized beverage into the hydrogen peroxide treated packaging including the fixed straw, and
g) Sealing of the packaging.

Surprisingly it was found that it is possible to achieve a microbiological safe and convenient packaging solution, wherein a straw is securely integrated inside the beverage packaging, by using above described process. It is especially possible to avoid state-of-the art hot-fill processes, resulting in an overall economic filling and packaging process, wherein the thermal load on the product is significantly reduced. In consequence, the same or even better microbiological safety levels and minimum storage lifes can be achieved and, at the same time, the packaged beverages comprise higher nutrient levels, based on the reduction of the thermal stress during packaging. In addition, it is possible to achieve better taste levels of the beverages, even at reduced sugar contents, rendering the product healthier compared to standardly processed beverages. The energy consumption of the overall process is much lower compared to the state of the art processes and further, some of the energy can even be recycled within this process. In hot fill processes the energy is lost, because it is technically difficult to cool the single packaging after filling. In the now proposed process it is possible to transfer heat from the beverage product stream before filling, because the filling temperature of the beverage into the packaging is not the microbiology determining step any more. Therefore, the energy costs are significantly reduced, i.e. in the range of approximately 50 % compared to a hot fill process of the same product output. Furthermore, better recyclable packaging material can be used, which cannot be processed under hot-fill conditions. The overall water consumption can be reduced, because it is not necessary to significantly cool the product after the filling process. An additional advantage is that the processing can be significantly faster, because the cooling time of the packaged beverage can be reduced. The cold filling is especially possible from 4-5°C up to ambient temperature. Furthermore, the microwave pasteurization results in a more homogeneous temperature profile throughout the beverage, leading to better microbiological kill rates and a more uniform product after pasteurization. The overall process is more environmentally friendly and at the same time a greener product comprising a better carbon footprint becomes available.

The filling temperature can be freely adjusted and can even be set to the necessary storage temperature of the packaged beverage. Therefore, no additional waiting period or other temperature adjusting means are necessary between packaging and storage. This keeps the overall process very fast and energy efficient. Furthermore, it is possible to re-formulate the beverage composition, because the beverage composition is usually adjusted with respect to the process. In hot-fill processes additional flavor carriers are sometimes added to the beverages in order to compensate the flavor loss in packaging. In the now proposed process this is unnecessary and in consequence healthier beverages can be offered, wherein the salt or sugar content is reduced compared to standard beverages being obtained by hot-fill processes. In addition, the proposed process is environmentally friendly, because no toxic chemicals are used in the process.

According to the invention a process for filling of beverage packaging is disclosed, wherein the process is especially suitable for industrial filling at high speeds. Beverages in the sense of this invention are all kinds of liquids suitable for human consumption. The process mainly concerns the filling of the beverage into the packaging including a straw and the necessary pre-steps on the packaging material and the liquid in order to achieve a microbiological safe product in the packaging. It is particularly not intended to provide a process, wherein the microbiological status of the beverage or the packaging is adjusted by physical means after the filling process.

The packaging according to the invention comprises an interior drinking straw inside the packaging. This means, that the beverage packaging also surrounds the drinking straw and the beverage and the drinking straw is accessible at the same time after only opening one packaging. The latter may be achieved for instance by tearing the beverage packaging at one point.

In step a) the beverage is pasteurized microwave assisted and in-line. This process step particularly excludes the use of pure thermal means (like steam or resistance) for beverage heating. The pasteurization is achieved by using high frequency microwaves, e.g. around approximately 2.450 MHz; for treatment of a beverage stream. The microwaves induce an even and fast thermal heating of the beverage and is able to reduce the microbiological load. This step may be achieved by passing the beverage stream through a microwave transparent pipe (e.g. based on Teflon), which is surrounded by one or more microwave generators. Based on the beverage flow speed, the desired temperature, the amount of microwave generators and their power settings, the resulting temperature of the beverage stream can be set. Suitable microwave heating times can be up to 30 seconds and suitable temperatures of the beverages can go from 10-15°C degrees up to 76-80 °C. Within these time and temperatures ranges a log reduction of 3-5 is achievable for the beverages.

In a second step b) the packaging is treated by Vaporized Hydrogen Peroxide (VHP). This means that the packaging material is contacted with vaporized hydrogen peroxide for a certain time period. The packaging material is either treated in the form of the final packaging or in the form of a pre-material, e.g. a film, which is converted to a pouch following the peroxide treatment. The efficiency of the Vaporized Hydrogen Peroxide treatment can principally be adjusted as a function of the vaporized hydrogen peroxide concentration and the contact time. Generally treatment times can be in the range of seconds. Such VHP treatment speeds enables the set-up of high throughput filling lines, wherein easily more than 100 packages/minute are producible. The hydrogen peroxide may be food grade H₂O₂ solution (35%). As a function of the packaging material and the treatment conditions principally sterile packaging material can be provided to the filling step. Nevertheless, in certain cases it might be sufficient not to achieve a completely aseptic material, but instead to achieve only a log reduction (e.g. 3-5) in microbiological counts of the packaging and the beverage, respectively. Also these situations are in the scope of this invention.

In step c) the straw is treated by Vaporized Hydrogen Peroxide. Surprisingly it was found that it is possible to obtain within suitable reaction times an essentially clean straw material. This is surprising, because straws exhibit an unsuitable geometry for the hydrogen peroxide diffusion and it was assumed that it is difficult to assure proper sterilization in the field of foodstuff without further technical measures. Nevertheless, it is possible to achieve either sterile or nearly microbiological clean material at moderate peroxide concentrations and reaction times.

In step d) the Vaporized Hydrogen Peroxide treated straw is assembled in the Vaporized Hydrogen Peroxide treated packaging. The assembly may be performed on a 1 to 1 basis in the meaning that one packaging and one straw is sterilized within the filling chamber and both are assembled. It is also feasible to perform the peroxide treatments on a number of packaging and/or straws and to store the vapor treated material in the meantime before the straw and the packaging is assembled. The assembly can be performed by known technical means and may usually be achieved by placing the straw in a certain position relative to the packaging.

In step e) the straw is fixed inside the packaging. The fixation of the straw inside of the packaging may be achieved by processes which are able to assure that the straw is attached to the packaging and keeps in position during the filling process. This may e.g. be achieved by pure physical forces between the packaging and the straw or by heat sealing the straw onto the packaging material.

In step f) the pasteurized beverage obtained in step a) is temperature adjusted and cold filled into the hydrogen peroxide treated packaging including the fixed straw. The temperature adjustment of the pasteurized beverage can be achieved by techniques known to the skilled artisan. In certain cases it can be performed by heat exchanging means like a plate heat exchanger. Suitably the temperature of the beverage is adjusted to a temperature from 5°C up to 20°C in order to maintain the valuable nutrients like the vitamins of the beverage intact. Furthermore, the adjustment of the temperature and especially the cold filling process will speed up the overall assembly and packaging process, because either different packaging material (e.g. with lower heat resistance properties) can be used or no extra waiting time has to be included in the process in order to achieve the right temperature at the different packaging steps.

Finally, the packaging including the beverage and the fixed straw is sealed in step g). The sealing of the packaging can be achieved by means known to the skilled artisan. This may for instance either be heat sealing of a packaging film or by screwing a cap or a septum onto the packaging. The only technical requirement can be seen in that the last step has to assure that the packaging inside is at least microbiologically separated from the outside.

In a preferred embodiment the Vaporized Hydrogen Peroxide treatment of the packaging and the straw in step b) and step c) is performed in-line and the Vaporized Hydrogen Peroxide treatment is a hydrogen peroxide dry gas process. A dry gas process means that the hydrogen peroxide is vaporized under conditions, wherein the vapor is essentially prevented from re-condensing. Therefore, it is possible to work with low hydrogen peroxide concentrations resulting in low costs. In addition, this dry gas process might reduce the negative impact of the sterilization treatment on the packaging material.

In a further characteristic of the process the microwave assisted pasteurization in step a) is performed at a temperature of ≥ 70°C and ≤ 100°C for a time period of ≥ 20 seconds and ≤ 150 seconds. Within this temperature and time range the beverages can be pasteurized in a fast and gentle manner, resulting in a better flavor and higher nutrient content after pasteurization.

In another aspect of the invention in step f) the energy obtained by the temperature adjustment of the beverage is used to adjust the beverage temperature before the microwave assisted pasteurization in step a). In order to obtain a cold fill process in most of the cases it is suitable to cool the beverage after the pasteurization step before filling the packaging. The excess heat can be transferred to a cooling liquid and the heated cooling liquid can be used to pre-heat the beverage before the microwave assisted pasteurization step. In this embodiment the overall energy needed for the microwave step is reduced, because some of the temperature is already provided by the pre-heated cooling liquid. This process exhibits lower energy costs at an uncompromising food safety level. Hence, this process is especially more efficient compared to a hot-fill HPP process and more environmentally friendly. In a preferred embodiment at least 20%, preferably 30 % of the energy needed in the pasteurization step is provided via heat exchange from later process stages.

In a further embodiment the cold filling is performed at a beverage temperature of ≥ 5°C and ≤ 25°C. Within this temperature range in step f) it is additionally possible that the end product is transferred to a warehouse storage without any time delays. For instance it is possible to load the product directly into a lorry. The product can be handled or packed into lower grade environmentally friendly carton packaging. The carton design will not suffer any dew point or temperature compatibility problems and a step towards an environmentally friendlier and cheaper packaging is possible. In addition, in comparison to a conventional hot filling process, wherein the filling is performed at ≥ 78°C, such process does not require water bath usage and cooling tunnels in order to reduce the temperature below e.g. ≤ 40°C.

In another characteristic of the process the packaging may be selected from the group consisting of pouches, flexible stand-up pouches, bottles, jars, sachets and cans. Especially this class of packaging material has been found suitable for the inventive process because the materials are compatible with the VHP integrated treatment. This results in long shelf lives of the packaged product even under non-chilling conditions.

Within a preferred aspect the packaging used in the disclosed packaging process is a pouch. Particularly pouches are compatible with the described process. This is mainly based on the fact that it is possible to separate the material sterilization from the pouch formation. It is for instance possible to sterilize a film laminate by the dry gas process and to form the pouch after the vapor treatment step. In this case a simple straight surface can be exposed to the hydrogen peroxide vapor. This avoids situations, wherein complicated pouch geometries has to be sterilized and extra care has to be taken to assure, that also deep pouch cavities get in sufficient contact with the peroxide gas. Therefore, the VHP gas treatment can be performed easier and at shorter process times.

In another embodiment of the invention the pouch may be made from a laminated film structure, wherein at least one film layer of the laminated film structure is selected from the group consisting of Linear Low Density Polyethylene (LLDPE), Metallocene Linear Low Density Polyethylene (MLLDPE), VLDPE (Very Low Density Polyethylene) or Metallocene Very Low Density Polyethylene (mVLDPE) films. This group of film material is very compatible with the proposed hydrogen peroxide sterilization step and also offers additionally advantages in the cold filling step. The surfaces of the film material can be sterilized with the Vaporized Hydrogen Peroxide very fast. The latter sealing step can be performed quicker compared to hot-fill processes, because film temperatures are lower and the sealing can be performed under different temperature conditions. This might lead to an overall improvement in the packaging output. Furthermore, these film materials are recyclable, increasing the ecological and environmental friendliness of the process.

Within another aspect of the invention the hydrogen peroxide dry gas process in step b) and c) is performed for a time period of ≥ 5 seconds and ≤ 100 seconds. This time period is sufficient to achieve excellent kill rates at fast processing times. It is further preferred to maintain in step b) and c) a temperature of ≥ 40°C and ≤ 50°C and to keep a contacting time period of ≥ 10 seconds and ≤ 25 seconds. In order to maintain sufficient hydrogen peroxide concentration it is possible to use hydrogen peroxide feeds of 1-12g/minute.

In a further aspect in step c) the hydrogen peroxide gas can be directed at least partly into the straw by active convection. In the case that packaging containing large beverage volumes are desired it might be necessary to also include long straws. In order to assure short packaging times it might be feasible to assist the hydrogen peroxide treatment especially of the straw by further blowing the vapor into the straw. This might increase the effective concentration of the peroxide per time unit. Therefore, the packaging line can be run faster without the risk of leaving the straw in non-sterile conditions. The active convection can for instance be achieved by a guided vapor stream, generated by a ventilator.

Within another characteristic the beverages can be selected from the group consisting of water, flavored water, milk, juices, fruit juice, smoothies. Especially these beverages are prone to degeneration in the course of a hot-fill process. This might be attributed to the nutrient composition and content of the listed beverages. Therefore, it is advantageous to use a cold-fill process, wherein the processing time at higher temperatures can be significantly reduced.

Within a preferred embodiment of the process, in step e) the fixation of the straw to the packaging can be achieved by a dynamic induction heating process. By using induction heating in the framework of a cold-fill process it is possible to keep the overall filling speed up and additionally provide some temperature nearby the straw. This may further increase the microbiological safety of the straw surrounding, without deteriorating the beverage quality.

In another aspect the straw may be made from PE, PP or mixtures thereof. Especially PE and/or PE can be used within the proposed sterilization process and deliver high log microbiological reductions rates even at very gentle hydrogen peroxide conditions and at short treatment times. In addition, no changes in the material properties are observed upon the hydrogen peroxide dry gas process.

Within a further aspect the straw may comprise a PE-layer and the pouch film may comprise a multi-layered structure at least comprising a polypropylene- (PP), an ethylene vinyl alcohol-(EVOH) and a polyethylene- (PE) layer. This means that the straw can be made from PE or may comprise a layered structure also comprising a PE-layer. Preferably, the straw may comprise an outer layer made from PE material. Inner layers may for instance be a paper layer or any other film material comprising a good recyclability. Surprisingly it was found that with such a layer structure or with a straw comprising only a single PE layer it is possible to achieve a high throughput process with an excellent microbiology. Such finding is not obvious, because PE straws usually comprise not the right thermal stability and bend upon extrusion and also in hot-fill processes. The bending of the straw is disadvantageous, because the bending hinders the sterilization process, especially in cases wherein gaseous sterilization means are used. The latter is the case in the inventive process. In addition, by using such straw structures it is possible to maintain high sealing speeds in the process and reliably and reproducibly induction heat seal the straw to the pouch material. Therefore, at high production rates a very low sealing failure level can be achieved by using a PE-straws, also reducing the production costs. In summary, the use of a PE straw is environmentally friendly and can increase the production rate of the process. A further preferred straw composition may comprise a 80:20 PE:PP blend. Such blend has also proven advantageous in production and, in addition, is a recyclable straw mix, belonging to recycle code 2 (UK) in the same group as food containers, dishware, compost bins, speed humps and gardening apparatus (compost bins, garden edging and plant pots). Such group is much better compared to standard pouches including straw applications, wherein the latter usually comprise aluminum layer structures, because code 2 material can readily be recycled to clothing or industrial fibres. The 80:20 mix straw material can also not be used in a hot-fill process, because it was found that based on the thermal load on the straw wither the straw material or the sealing will fail.

The pouch material can advantageously be a multi layered film material, wherein the film material does not comprise an aluminum layer. It was surprisingly found that a PP:EVOH:PE delivers in combination with the inventive production process excellent sterilizability, product shelf live and high production throughputs. Such finding is surprising, because usually the performance of this layer combination in standard hot-fill processes is very low, exhibiting high failure rates and very bad sealing behavior. Therefore, the inventive process is able to process a greener and better recyclable material and the same time higher processing rates compared to standard hot-fill processes. Especially, it was found that the water, flavour and UV barrier properties of this film material after sealing and the overall oxygen, oil and chemical barrier properties are excellent. The sealing rates and the sealing quality by induction sealing in combination with a straw also comprising a PE layer are also very good, resulting in a streamlined production process. In addition, this pouch material enables superior laser cutting properties and it is possible to precisely perform a laser cutting of the pouch cap above the straw in order to guide only a partial tearing of the pouch cap. This is also possible at high processing speeds and result in an application, wherein after tearing a part of the pouch cap remains on the pouch. In consequence, only one pouch piece has to be recycled and the number of waste is reduced. It is further within the scope of the invention to also provide a system for filling of beverage packaging comprising an interior drinking straw at least comprising
a) a microwave assisted pasteurization unit for pasteurization of beverages
b) a Vaporized Hydrogen Peroxide treatment unit for treatment of the packaging material and the straw,
c) a heat exchange unit for temperature adjustment of the pasteurized beverage before filling,
d) a cold filling unit, a straw assembling unit, a straw fixing unit, and
e) an induction sealing unit for sealing the packaging.

This system is suited to perform the above described filling process and hence all advantages and process conditions described for the inventive process shall be deemed disclosed and applicable for the inventive usable system. The overall system can also be described as a MSCF-system (Microwave Sterilize Cold Fill-system). Especially this system avoids a pre- or after burning effect on the liquid products, which may typically appear when the product is passing a conventional pasteurization system. By using such systems a fast and reliable beverage packaging comprising an interior drinking straw can be obtained, which in turn results in an efficient reduction of packaging costs. Furthermore, this system is also able to deliver the same amounts of beverage packaging at significantly reduced energy costs. The latter is especially true in comparison to state of the art hot-fill process. Therefore, the CO2-load for the packaging is smaller compared to the hot-fill process, rendering the overall process "greener".

### FIGURES

A preferred embodiment of the invention has been chosen for purposes of illustration and description and is shown in the accompanying drawings, wherein:
FIG. 1 is a schematic front view of a possible pouch packaging (1) including a straw (2), which can be used within the inventive packaging process;
FIG. 2 is a schematic side view of a pouch packaging (1) including a straw (not shown), which can be used within the inventive packaging process;
FIG. 3 is a schematic representation of the inventive process;
FIG. 4 is a schematic front view of a possible pouch packaging (1) including a drinking straw (2), which can be used within the inventive packaging process; and
FIG. 5 is a schematic representation of a possible film material (5) sealing pattern to produce a straw (2) from a film material (4) by heat sealing.

### DETAILED DESCRIPTION OF FIGURES

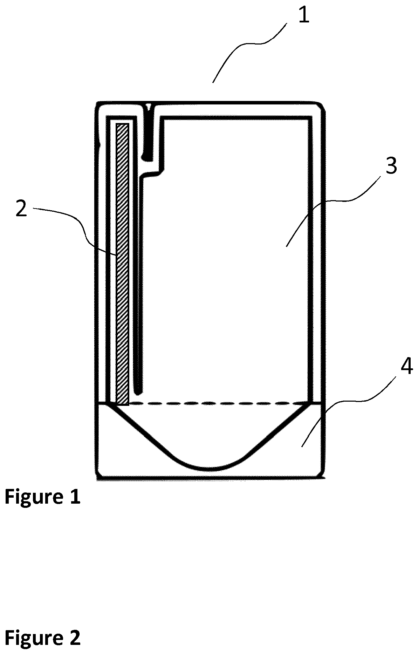

Figure 1 exhibits a pouch packaging (1) including a drinking straw (2). The drinking straw (2) is integrated within the pouch packaging (1) and extends into the beverage (3), which is included in the pouch packaging. The pouch (1) is made from film material (4). This packaging enables a safe and convenient application.

Figure 2 displays a side view of the drinking pouch (1). The drinking straw (2) inside the packaging is not shown.

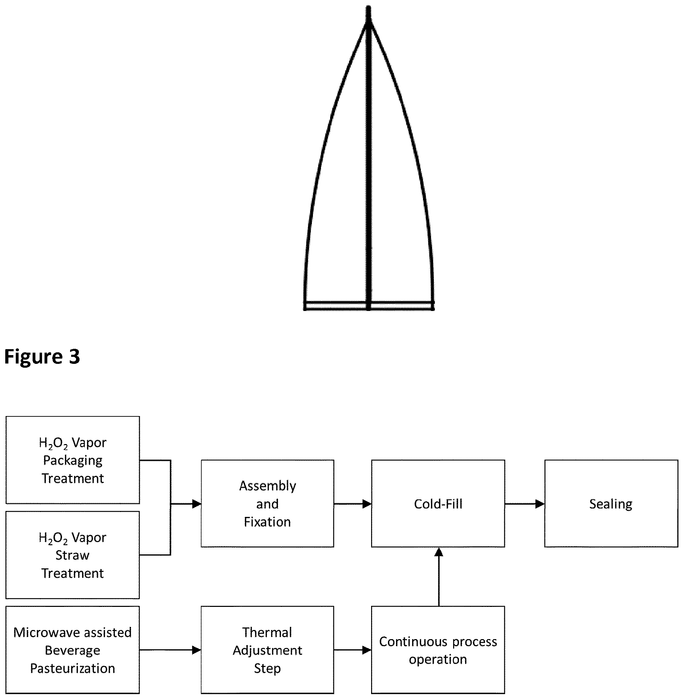

Figure 3 is a schematic representation of the inventive process. In a first step the beverage is pasteurized using microwave assisted pasteurization. The output of the pasteurization step is fed into a thermal adjustment step, wherein the temperature of the beverage is adjusted by heat exchange means. The temperature controlled, pasteurized beverage is either directly guided to the cold-fill process or the pasteurized liquid may be transferred to a buffer. Both possibilities are able to assure a continuous production process. The beverage packaging and the straw are each independent and independently from the beverage treated with hydrogen peroxide VHP dry gas in order to achieve a sterilization effect on the packaging material and the straw. The packaging material and the straw are assembled and the straw is fixed in the assembled position. This step is followed by cold-filling of the pasteurized and thermally adjusted beverage into the assembled packaging. In the last process step the packaging including the straw and the beverage is sealed.

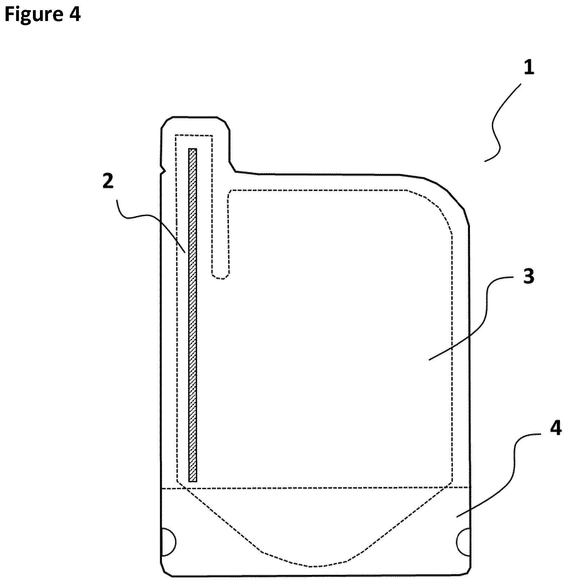

Figure 4 displays another front view of a possible pouch (1) packaging which can be processed according to the invention. The packaging (1) comprises a drinking straw (2) inside of the packaging (1). The straw is in direct contact with the beverage (3), complicating the microbiological safety situation. It is additionally displayed, that after the packaging is partially teared open at the straw (2) some part of the cap may still remain at the packaging. Such design helps reducing the number of waste material for disposal.

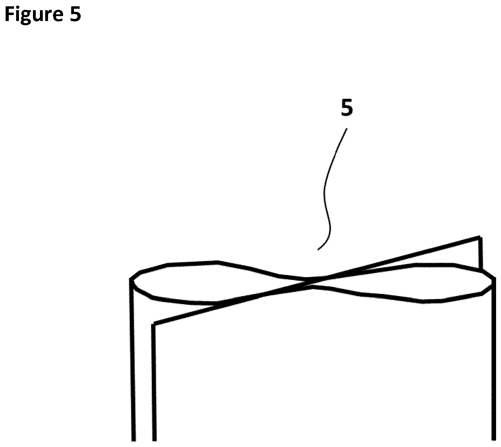

Figure 5 shows a possible folding pattern (5) for heat sealing a straw (2) from a film material (4). The folded film creates a figure of eight straw (2) and can be heat-sealed in the center into this conformation. Such straw set-up (2) enables processing of the complete packaging (1) from one film material (4), only. This creates a packaging which is easier to recycle. It is possible for instance to use a PE-film or a PE-film laminate to fold and create a straw and heat seal the film.

### REFERENCE NUMERALS

- 1: Pouch packaging
- 2: Drinking straw
- 3: Beverage
- 4: Material
- 5: drinking straw figure of 8 configuration

## Claims

1. Process for filling of beverage packaging (1) comprising an interior drinking straw (2), **characterized in that** the filling process at least comprises the steps of
a) in-line microwave assisted pasteurization of the beverage (3),
b) Vaporized Hydrogen Peroxide treatment of the packaging (1),
c) Vaporized Hydrogen Peroxide treatment of the straw (2),
d) Assembly of the Vaporized Hydrogen Peroxide treated straw (2) in the Vaporized Hydrogen Peroxide treated packaging (4),
e) Fixation of the straw (2) inside the packaging (4),
f) temperature adjustment of the pasteurized beverage (3) of step a) and cold filling of the pasteurized beverage (3) into the hydrogen peroxide treated packaging (4) including the fixed straw (2), and
g) Sealing of the packaging (4).

2. Process according to claim 1, wherein the Vaporized Hydrogen Peroxide treatment of the packaging (4) and the straw (2) in step b) and step c) is performed in-line and the Vaporized Hydrogen Peroxide treatment is a hydrogen peroxide dry gas process.

3. Process according to any of the preceding claims, wherein the microwave assisted pasteurization in step a) is performed at a temperature of ≥ 70°C and ≤ 100°C for a time period of ≥ 20 seconds and ≤ 150 seconds.

4. Process according to any of the preceding claims, wherein in step f) the energy obtained by the temperature adjustment of the beverage is used to adjust the beverage temperature before the microwave assisted pasteurization in step a).

5. Process according to any of the preceding claims, wherein the cold filling is performed at a beverage temperature of ≥ 5°C and ≤ 25°C.

6. Process according to any of the preceding claims, wherein the packaging (1) is selected from the group consisting of pouches, flexible stand-up pouches, bottles, jars, sachets and cans.

7. Process according to any of the preceding claims, wherein the packaging (1) is a pouch.

8. Process according to claim 7, wherein the pouch (1) is made from a laminated film structure, wherein at least one film layer of the laminated film structure is selected from the group consisting of PE, PP, EVOH, Linear Low Density Polyethylene, LLDPE, Metallocene Linear Low Density Polyethylene, MLLDPE, VLDPE, Very Low Density Polyethylene, or Metallocene Very Low Density Polyethylene, m VLDPE, films.

9. Process according to claim 2, wherein the hydrogen peroxide dry gas process in step b) and c) is performed for a time period of ≥ 5 seconds and ≤ 100 seconds.

10. Process according to claim 9, wherein in step c) the hydrogen peroxide gas is directed at least partly into the straw (2) by active convection.

11. Process according to any of the preceding claims, wherein the beverages (3) are selected from the group consisting of water, flavored water, milk, juices, fruit juice and smoothies.

12. Process according to any of the preceding claims, wherein in step e) the fixation of the straw (2) to the packaging is achieved by a dynamic induction heating process.

13. Process according to any of the preceding claims, wherein the straw (2) is made from PE, PP or mixtures thereof.

14. Process according to any of the preceding claims, wherein the straw (2) comprises a PE-layer and the pouch film comprises a multi-layered structure at least comprising a polypropylene- (PP), an ethylene vinyl alcohol- (EVOH) and a polyethylene- (PE) layer.

15. System for filling of beverage packaging (1) comprising an interior drinking straw (2) at least comprising
a) a microwave assisted pasteurization unit for pasteurization of beverages (3),
b) a Vaporized Hydrogen Peroxide sterilization unit for sterilization of the packaging material (4) and the straw (2),
c) a heat exchange unit for temperature adjustment of the pasteurized beverage (3) before filling,
d) a cold filling unit, means for assembly of the treated straw in the packaging, means for fixation of the straw inside the packaging, and
e) an induction sealing unit for sealing of the packaging (1).

## Patentansprüche

1. Verfahren zum Füllen von Getränkeverpackungen (1), die einen internen Trinkhalm (2) umfassen, **dadurch gekennzeichnet, dass** das Füllverfahren wenigstens die Schritte umfasst:
a) mikrowellenunterstützte Inline-Pasteurisation des Getränks (3),
b) Behandlung der Verpackung (1) mit verdampftem Wasserstoffperoxid,
c) Behandlung des Trinkhalms (2) mit verdampftem Wasserstoffperoxid,
d) Anordnen des mit verdampftem Wasserstoffperoxid behandelten Trinkhalms (2) in der mit verdampftem Wasserstoffperoxid behandelten Verpackung (4),
e) Befestigen des Trinkhalms (2) im Inneren der Verpackung (4),
f) Temperatureinstellung des pasteurisierten Getränks (3) aus Schritt a) und Kaltfüllen des pasteurisierten Getränks (3) in die mit Wasserstoffperoxid behandelte Verpackung (4), die den befestigten Trinkhalm (2) enthält, und
g) Verschließen der Verpackung (4).

2. Verfahren gemäß Anspruch 1, wobei die Behandlung der Verpackung (4) und des Trinkhalms (2) mit verdampftem Wasserstoffperoxid bei Schritt b) und Schritt c) inline durchgeführt wird und die Behandlung mit verdampftem Wasserstoffperoxid ein Wasserstoffperoxid-Trockengasverfahren ist.

3. Verfahren gemäß einem der vorstehenden Ansprüche, wobei die mikrowellenunterstützte Pasteurisation bei Schritt a) bei einer Temperatur von ≥ 70 °C und ≤ 100 °C über einen Zeitraum von ≥ 20 Sekunden und ≤ 150 Sekunden durchgeführt wird.

4. Verfahren gemäß einem der vorstehenden Ansprüche, wobei bei Schritt f) die durch die Temperatureinstellung des Getränks erhaltene Energie verwendet wird, um die Getränketemperatur vor dem Schritt a) der mikrowellenunterstützten Pasteurisation einzustellen.

5. Verfahren gemäß einem der vorstehenden Ansprüche, wobei die Kaltfüllung bei einer Getränketemperatur von ≥ 5 °C und ≤ 25 °C durchgeführt wird.

6. Verfahren gemäß einem der vorstehenden Ansprüche, wobei die Verpackung (1) ausgewählt ist aus der Gruppe bestehend aus Beuteln, flexiblen Standbeuteln, Flaschen, Krügen, Tüten und Dosen.

7. Verfahren gemäß einem der vorstehenden Ansprüche, wobei die Verpackung (1) ein Beutel ist.

8. Verfahren gemäß Anspruch 7, wobei der Beutel (1) aus einer laminierten Filmstruktur besteht, wobei wenigstens eine Filmschicht der laminierten Filmstruktur ausgewählt ist aus der Gruppe bestehend aus PE, PP, EVOH, linearem Polyethylen mit niedriger Dichte, LLDPE, linearem Metallocen-Polyethylen mit niedriger Dichte, MLLDPE, VLDPE, Polyethylen mit sehr niedriger Dichte oder Metallocen-Polyethylen mit sehr niedriger Dichte, mVLDPE, Filmen.

9. Verfahren gemäß Anspruch 2, wobei das Wasserstoffperoxid-Trockengasverfahren bei Schritt b) und c) über einen Zeitraum von ≥ 5 Sekunden und ≤ 100 Sekunden durchgeführt wird.

10. Verfahren gemäß Anspruch 9, wobei bei Schritt c) das Wasserstoffperoxidgas wenigstens teilweise durch aktive Konvektion in den Trinkhalm (2) gelenkt wird.

11. Verfahren gemäß einem der vorstehenden Ansprüche, wobei die Getränke (3) ausgewählt sind aus der Gruppe bestehend aus Wasser, aromatisiertem Wasser, Milch, Säften, Fruchtsaft und Smoothies.

12. Verfahren gemäß einem der vorstehenden Ansprüche, wobei bei Schritt e) das Befestigen des Trinkhalms (2) an der Verpackung durch ein dynamisches Induktionsheizverfahren durchgeführt wird.

13. Verfahren gemäß einem der vorstehenden Ansprüche, wobei der Trinkhalm (2) aus PE, PP oder Gemischen davon besteht.

14. Verfahren gemäß einem der vorstehenden Ansprüche, wobei der Trinkhalm (2) eine PE-Schicht umfasst und der Beutelfilm eine mehrschichtige Struktur umfasst, die wenigstens eine Polypropylen- (PP), eine EthylenVinylalkohol- (EVOH) und eine Polyethylenschicht (PE) umfasst.

15. System zum Füllen von Getränkeverpackungen (1), die einen internen Trinkhalm (2) umfassen, wenigstens umfassend:
a) eine mikrowellenunterstützte Pasteurisationseinheit zur Pasteurisation von Getränken (3),
b) eine Einheit zur Sterilisation mit verdampftem Wasserstoffperoxid zur Sterilisation des Verpackungsmaterials (4) und des Trinkhalms (2),
c) eine Wärmetauscheinheit zur Temperatureinstellung des pasteurisierten Getränks (3) vor dem Abfüllen,
d) eine Kaltfülleinheit, eine Einrichtung zum Anordnen des behandelten Trinkhalms in der Verpackung, eine Einrichtung zum Befestigen des Trinkhalms im Inneren der Verpackung und
e) eine Induktionssiegeleinheit zum Verschließen der Verpackung (1).

## Revendications

1. Procédé pour le remplissage d'un emballage (1) pour boisson comprenant une paille à boire intérieure (2), **caractérisé en ce que** le procédé de remplissage comprend au moins les étapes de
a) pasteurisation assistée par des micro-ondes en ligne de la boisson (3),
b) traitement au Peroxyde d'Hydrogène Vaporisé de l'emballage (1),
c) traitement au Peroxyde d'Hydrogène Vaporisé de la paille (2),
d) assemblage de la paille (2) traitée au Peroxyde d'Hydrogène Vaporisé dans l'emballage (4) traité au Peroxyde d'Hydrogène Vaporisé,
e) fixation de la paille (2) à l'intérieur de l'emballage (4),
f) ajustement de la température de la boisson (3) pasteurisée de l'étape a) et remplissage à froid de la boisson (3) pasteurisée dans l'emballage (4) traité au peroxyde d'hydrogène comprenant la paille (2) fixée, et
g) scellement de l'emballage (4).

2. Procédé selon la revendication 1, le traitement au Peroxyde d'Hydrogène Vaporisé de l'emballage (4) et de la paille (2) dans l'étape b) et dans l'étape c) étant réalisé en ligne et le traitement au Peroxyde d'Hydrogène Vaporisé étant un procédé au gaz sec de peroxyde d'hydrogène.

3. Procédé selon l'une quelconque des revendications précédentes, la pasteurisation assistée par des micro-ondes dans l'étape a) étant réalisée à une température de ≥ 70 °C et ≤ 100 °C pendant une période de temps de ≥ 20 secondes et ≤ 150 secondes.

4. Procédé selon l'une quelconque des revendications précédentes, dans lequel dans l'étape f) l'énergie obtenue par l'ajustement de la température de la boisson est utilisée pour ajuster la température de la boisson avant la pasteurisation assistée par des micro-ondes dans l'étape a).

5. Procédé selon l'une quelconque des revendications précédentes, le remplissage à froid étant réalisé à une température de boisson de ≥ 5 °C et ≤ 25 °C.

6. Procédé selon l'une quelconque des revendications précédentes, l'emballage (1) étant choisi dans le groupe constitué par des poches, des poches flexibles à maintien vertical, des bouteilles, des pots, des sachets et des canettes.

7. Procédé selon l'une quelconque des revendications précédentes, l'emballage (1) étant une poche.

8. Procédé selon la revendication 7, la poche (1) étant composée d'une structure de film stratifiée, au moins une couche de film de la structure de film stratifiée étant choisie dans le groupe constitué par des films de PE, de PP, d'EVOH, de Polyéthylène Linéaire à Basse Densité, LLDPE, de Polyéthylène Linéaire à Basse Densité de Métallocène, MLLDPE, de VLDPE, Polyéthylène à Très Basse Densité, et de Polyéthylène à Très Basse Densité de Métallocène, mVLDPE.

9. Procédé selon la revendication 2, le procédé au gaz sec de peroxyde d'hydrogène dans l'étape b) et c) étant réalisé pendant une période de temps de ≥ 5 secondes et ≤ 100 secondes.

10. Procédé selon la revendication 9, dans lequel dans l'étape c) le gaz de peroxyde d'hydrogène est dirigé au moins partiellement dans la paille (2) par convection active.

11. Procédé selon l'une quelconque des revendications précédentes, les boissons (3) étant choisies dans le groupe constitué par de l'eau, de l'eau aromatisée, du lait, des jus, un jus de fruits et des smoothies.

12. Procédé selon l'une quelconque des revendications précédentes, dans lequel dans l'étape e) la fixation de la paille (2) à l'emballage est réalisée par un procédé de chauffage par induction dynamique.

13. Procédé selon l'une quelconque des revendications précédentes, la paille (2) étant composée de PE, de PP, ou de mélanges correspondants.

14. Procédé selon l'une quelconque des revendications précédentes, la paille (2) comprenant une couche de PE et le film de la poche comprenant une structure multicouche comprenant au moins une couche de polypropylène (PP), une couche d'éthylène-alcool vinylique (EVOH) et une couche de polyéthylène (PE).

15. Système pour le remplissage d'un emballage (1) pour boisson comprenant une paille à boire intérieure (2) comprenant au moins :
a) une unité de pasteurisation assistée par des micro-ondes pour la pasteurisation de boissons (3),
b) unité de stérilisation au Peroxyde d'Hydrogène Vaporisé pour la stérilisation du matériau d'emballage (4) et de la paille (2),
c) une unité d'échange de chaleur pour l'ajustement de la température de la boisson (3) pasteurisée avant le remplissage,
d) une unité de remplissage à froid, un moyen pour l'assemblage de la paille traitée dans l'emballage, un moyen pour la fixation de la paille à l'intérieur de l'emballage, et
e) une unité de scellement par induction pour le scellement de l'emballage (1).
